# EUROPÄISCHE PATENTSCHRIFT

(11) **EP 0 697 398 B1**
(45) Veröffentlichungstag und Bekanntmachung des Hinweises auf die Patenterteilung: **29.10.1997**
(21) Anmeldenummer: 94113003.1
(22) Anmeldetag: 19.08.1994
(51) Int. Cl.: C07C 231/08, C07D 207/267, C07D 223/10

(54) **Verfahren zur Herstellung von N-Alkenylcarbonsäureamiden**
Process for the preparation of N-alkenyl carboxylic-amides
Procédé pour la préparation d'amides d'acide carboxylique N-alcényl

(43) Veröffentlichungstag der Anmeldung: 21.02.1996
(73) Patentinhaber: BASF Aktiengesellschaft, 67063 Ludwigshafen (DE)
(72) Erfinder: Rühl, Thomas Dr., 67227 Frankentahl (DE); Henkelmann, Jochem Dr., 68165 Mannheim (DE); Heider, Marc Dr., 67433 Neustadt (DE)

(56) Entgegenhaltungen:
- EP-A- 0 350 666
- DE-A- 2 725 379
- US-A- 5 023 375
- DATABASE WPI Week 7223, Derwent Publications Ltd., London, GB; AN 72-37610T & JP-B-47 020 011 (TOA GOSEI CHEMICAL IND CO)

## Beschreibung

Die vorliegende Erfindung betrifft ein neues Verfahren zur Herstellung von N-Alkenylcarbonsäureamiden der allgemeinen Formel I 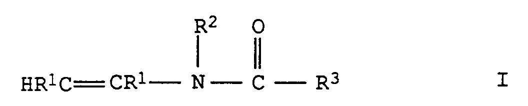 in der mindestens einer der Reste R¹ Wasserstoff bedeutet und der zweite Rest R¹ für Wasserstoff oder eine C₁-C₄-Alkylgruppe steht der Rest R² für einen aliphatischen, cycloaliphatischen, araliphatischen oder aromatischen Rest steht, der mit dem Rest R³ zu einem 3- bis 10-gliedrigen Brückenglied verbunden sein kann, und der Rest R³ für Wasserstoff, einen aliphatischen, cycloaliphatischen oder aromatischen Rest steht, aus einem Carbonsäurealkenylester der allgemeinen Formel II 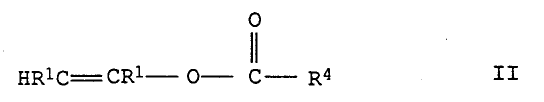 in der R¹ die oben angegebene Bedeutung hat und R⁴ für Wasserstoff, einen aliphatischen, cycloaliphatischen oder aromatischen Rest steht, und einem Carbonsäureamid der allgemeinen Formel III 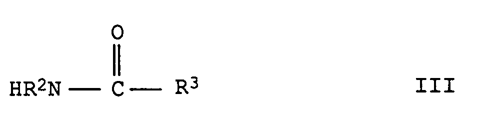 in der die Reste R² und R³ die oben angegebene Bedeutung haben.

Die Verfahrensprodukte der Formel I sind gesuchte Zwischenprodukte. N-Alkenylcarbonsäureamide können in bekannter Weise polymerisiert werden und anschließend durch Hydrolyse in die entsprechenden Polyvinylamine überführt werden. Diese Polymere, insbesondere Polyvinylpyrrolidon, dienen beispielsweise als Waschhilfsmittel, als Hilfsstoffe in kosmetischen und medizinischen Salben sowie zur Stabilisierung und zur Klärfiltration von Bieren und Fruchtsäften.

Reppe et al. lehren die Herstellung von N-Vinylcarbonsäureamiden durch Umsetzung von Carbonsäureamiden mit Acetylen unter Druck in Gegenwart von Basen (Liebigs Ann. Chem., 601 (1956) 82;

DE-A 12 45 939). Der Umgang mit Acetylen erfordert einen großen technischen Aufwand.

Die DE-A 23 36 977, DE-A 12 28 246 und DE-A 32 37 309 betreffen die Herstellung von substituierten N-Alkylamiden als Vorstufen für N-Vinylamide.

Diese Verfahren zur Herstellung der Vinylamide sind zweistufig und erfordern einen thermischen Eliminierungsschritt. Sie sind somit technisch relativ aufwendig und führen aufgrund von Verlusten an Wertprodukt bei den erforderlichen hohen Reaktionstemperaturen nur zu unbefriedigenden Gesamtausbeuten.

Die JP-A 72/20011 lehrt ein Verfahren zur Herstellung von N-Vinylpyrrolidon und N-Vinylcaprolactam durch Vinylierung der Lactame mit einem Vinylester in Gegenwart einer Säure und eines Quecksilbersalzes. Abgesehen von der aus toxikologischen Gründen unerwünschten Verwendung von Quecksilberverbindungen führt die Gegenwart von Säuren immer zu einer teilweisen Polymerisation des Verfahrensproduktes, was sich in einer unbefriedigenden Gesamtausbeute widerspiegelt.

Die DE-A 27 25 379 betrifft ein Verfahren zur Herstellung von N-Alkenylverbindungen aus Alkenylestern und N-substituierten Carbonsäureamiden in Gegenwart von Metallen der Platingruppe, insbesondere Palladium, als Katalysator. In technischen Verfahren kommt es dabei zur Reduktion des Katalysators, so daß es erforderlich wird, die teueren Edelmetallverbindungen nachzudosieren.

Es bestand daher die Aufgabe, ein Verfahren bereitzustellen, das die genannten Nachteile bekannter Verfahren vermeidet.

Demgemäß wurde das oben definierte Verfahren zur Herstellung von N-Alkenylcarbonsäureamiden der Formel I gefunden, das dadurch gekennzeichnet ist, daß man die Ausgangsverbindungen in Gegenwart einer Base umsetzt.

Die nachstehende Reaktionsgleichung verdeutlicht das beanspruchte Verfahren am Beispiel der Herstellung von N-Vinylpyrrolidon aus Vinylformiat und Pyrrolidon in Gegenwart von Triethylamin (Et = Ethyl):

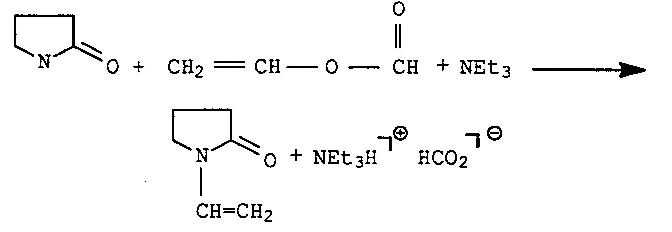

Im erfindungsgemäßen Verfahren wird formal eine Vinylgruppe aus einem Carbonsäurevinylester der Formel II auf ein Carbonsäureamid der Formel III übertragen.

Die Vinylgruppe der Ester der Formel II kann einen C₁-C₄-Alkylrest wie Methyl, Ethyl, n-Propyl, iso-Propyl und n-Butyl tragen, bevorzugt sind jedoch Verbindungen, in denen die Reste R¹ für Wasserstoff stehen. Der Rest R⁴ in den Estern der Formel II steht für aliphatische Reste wie Alkyl- und Alkenylgruppen, die vorzugsweise 1 bis 40 Kohlenstoffatome tragen und geradkettig oder verzweigt sein können. Besonders bevorzugt sind C₁-C₂₀-Alkylgruppen wie Methyl, Ethyl, n-Propyl, n-Butyl, tert.-Butyl, Neodecyl und Stearyl. Weiterhin kann R⁴ für cycloaliphatische Reste, vorzugsweise mit 4 - 7 Kohlenstoffatomen, stehen, z.B. Cyclopentyl und Cyclohexyl. Auch aromatische Reste wie Phenyl und Naphthyl kommen in Betracht, wobei diese unter den Reaktionsbedingungen inerte Substituenten wie Halogen, Alkoxy und Alkyl tragen können. Besonders bevorzugt steht R⁴ außerdem für Wasserstoff.

Als Ausgangsverbindungen der Formel II seien genannt: Vinylformiat, Vinylacetat, Vinylpropionat, Vinylstearat, Vinylpivalat und 4-tert.-Butylbenzoesäurvinylester.

Die Verbindungen der Formel II sind im Handel erhältlich oder nach bekannten Methoden herstellbar, beispielsweise durch Addition von Carbonsäuren an Acetylen oder durch Acetoxilierung von Ethylen (Weissermel u. Arpe, Industrielle Organische Chemie, 2. Auflage, 1978, Verlag Chemie, S. 217ff).

Der Rest R² in den Carbonsäureamiden der Formel III steht für aliphatische Reste wie Alkylgruppen, die bevorzugt 1 bis 10 Kohlenstoffatome tragen und geradkettig oder verzweigt sind. Besonders bevorzugt sind C₁-C₄-Alkylgruppen wie Methyl, Ethyl, n-Propyl und n-Butyl.

R² kann auch für einen cycloaliphatischen Rest wie Cyclohexyl, einen aromatischen Rest wie Phenyl und einen araliphatischen Rest wie Benzyl stehen. Der Rest R² kann mit dem Rest R³ weiterhin ein 3- bis 10-gliedriges Brückenglied formen, wovon 3- bis 7-gliedrige Alkylenbrückenglieder bevorzugt sind.

Für den Rest R³ der Amide der Formel III gilt das oben zum Rest R⁴ gesagte analog. Als Ausgangsverbindungen seien genannt: Pyrrolidon, Caprolactam, Piperidon, N-Methylacetamid, N-Phenylacetamid und N-Methylbenzoylamid.

Auch diese Verbindungen sind im Handel oder nach bekannten Methoden erhältlich, z.B. durch Umsetzung von Carbonsäuren und primären Aminen unter Wasserabspaltung.

Bevorzugte Verfahrensprodukte sind N-Vinylpyrrolidon und N-Vinylcaprolactam.

Die Ausgangsverbindungen werden in Gegenwart einer Base, vorzugsweise einer Brönsted-Base, umgesetzt. Dafür kommen sowohl anorganische wie organische Basen in Betracht. Dabei handelt es sich im einzelnen um Carbonate und Hydrogencarbonate der Alkali- und Erdalkalimetalle wie Natriumcarbonat, Kaliumcarbonat und Natriumhydrogencarbonat, quartäre Ammoniumcarbonate wie Tetramethylammoniumcarbonat, Amide wie Alkalimetallamide, beispielsweise Natriumamid und Kaliumamid, Hydroxide wie Alkalimetallhydroxide, beispielsweise Lithiumhydroxid, Natriumhydroxid und Kaliumhydroxid, Alkalimetallcarboxylate wie Natriumacetat, Alkoholate wie Alkalimetallalkoholate, beispielsweise Natriummethanolat, Natriumethanolat, Kaliummethanolat und Kalium-tert.-butanolat. Kaliumhydroxid kann auch zusammen mit Kronenethern wie 18-Krone-6 verwendet werden.

Als Basen kommen weiterhin Amine wie Ammoniak, primäre, sekundäre und tertiäre Amine in Betracht, von denen die tertiären bevorzugt sind. Die Amine können aliphatische oder aromatische Reste tragen, beispielsweise Trialkylamine wie Trioctylamin, Ethyldiisopropylamin, Diethylisopropylamin, Dimethylcyclohexylamin, Triethylamin, außerdem cyclische Amine wie 2,2,6,6-Tetramethylpiperidin, 1,4-Diazabicyclo[2.2.2]octan, 1,8-Diazabicyclo[5.4.0]undec-7-en, aliphatische und aromatische Reste tragende Amine wie 1,8-Bis(dimethylamino)-naphthalin und 4-Dimethylaminopyridin und heterocyclische Amine wie N-Alkylimidazole und N-Arylimidazole. Weiterhin kommen Amide wie Dialkylcarbonsäureamide, z.B. Dibutylformamid, in Betracht. Das erfindungsgemäße Verfahren läßt sich auch in Gegenwart von basischen Ionenaustauschern, die in der Regel aus sulfonierten Styrol-Divinylbenzol-Copolymerisaten bestehen wie Amberlite®, Lewatit® und Puralit® , und in Gegenwart von basischen Zeolithen wie Hydrotalcit durchführen.

Pro Äquivalent Amid der Formel III können 0,1 bis 10, vorzugsweise 1 bis 1,2 Äquivalente des Esters der Formel II eingesetzt werden.

Die Basenmenge kann 0,1 bis 3 Äquivalente, bevorzugt 0,2 bis 1 Äquivalent pro Äquivalent Amid der Formel III betragen.

Obwohl die Umsetzung bevorzugt ohne Lösungsmittel ausgeführt wird, kann jedoch ein Lösungsmittel zugesetzt werden, z.B. aprotische Lösungsmittel wie Ether, z.B. Tetrahydrofuran, aromatische Kohlenwasserstoffe wie Toluol und Xylol, Ketone wie Aceton, weiterhin Acetonitril, Hexamethylphosphorsäuretriamid, Sulfolan, Dimethylsulfoxid, Harnstoffe wie N,N'-Dimethylethylen-, N,N'-Dimethylpropylenharnstoff und Tetrabutylharnstoff. Die Menge liegt im allgemeinen bei 10 bis 30 Gew.-%, bezogen auf den Gesamtansatz.

Die Reaktionstemperatur liegt in der Regel bei 0 bis 150°C, bevorzugt bei 20 bis 80°C. Bevorzugt wird bei Normaldruck gearbeitet, es ist aber auch möglich, bei 0,01 bis 10 bar Reaktionsdruck zu arbeiten.

Die Reaktion kann kontinuierlich und diskontinuierlich ausgeführt werden. So können die Ausgangsverbindungen und die Base in einen Rührkessel gegeben und darin umgesetzt werden, wobei die Reihenfolge der Zugabe der Einzelkomponenten keinen erkennbaren Einfluß auf die Reaktion hat. Es ist auch möglich, die Ausgangsverbindungen und die Base in einem Rohrreaktor in Riesel- oder Sumpffahrweise umzusetzen. Es hat sich als vorteilhaft erwiesen, die Reaktion in einem Strahldüsenreaktor vorzunehmen.

In der Regel ist die Reaktion nach 5 Minuten bis 8 Stunden beendet.

Das so erhaltene Reaktionsgemisch kann in an sich bekannter Weise aufgearbeitet werden. Im allgemeinen wird das Produkt destillativ abgetrennt. Der Destillationssumpf kann zur Freisetzung der organischen Basen aus den bei der Reaktion anfallenden Salzen mit Laugen wie Natronlauge versetzt werden. Die eingesetzten Basen können anschließend extraktiv oder destillativ isoliert werden. Werden in der erfindungsgemäßen Umsetzung leichtflüchtige salzartige Verbindungen wie Formiate tertiärer Ammoniumverbindungen gebildet, können diese auch destillativ aufgearbeitet und in die entsprechenden Amine überführt werden. Die jeweils abgetrennten Basen können in die Reaktion zurückgeführt werden.

Das erfindungsgemäße Verfahren erlaubt die einstufige Herstellung von N-Alkenylamiden aus leicht zugänglichen Vorprodukten. Die Reaktion läßt sich technisch einfach gestalten und läuft unter milden Reaktionstemperaturen ab. Sie führt weiterhin zu einer hohen Ausbeute an den Verfahrensprodukten.

### Beispiele

### Allgemeine Reaktionsdurchführung

A mol eines Esters der Formel II, b mol eines Amids der Formel III und c mol Base wurden bei x°C und 1 bar zum Produkt I umgesetzt. Der Verlauf der Reaktion wurde gaschromatographisch verfolgt.

Die Ausbeute wurde gaschromatographisch bestimmt.

In der folgenden Tabelle sind Einzelheiten zu den Umsetzung angegeben:

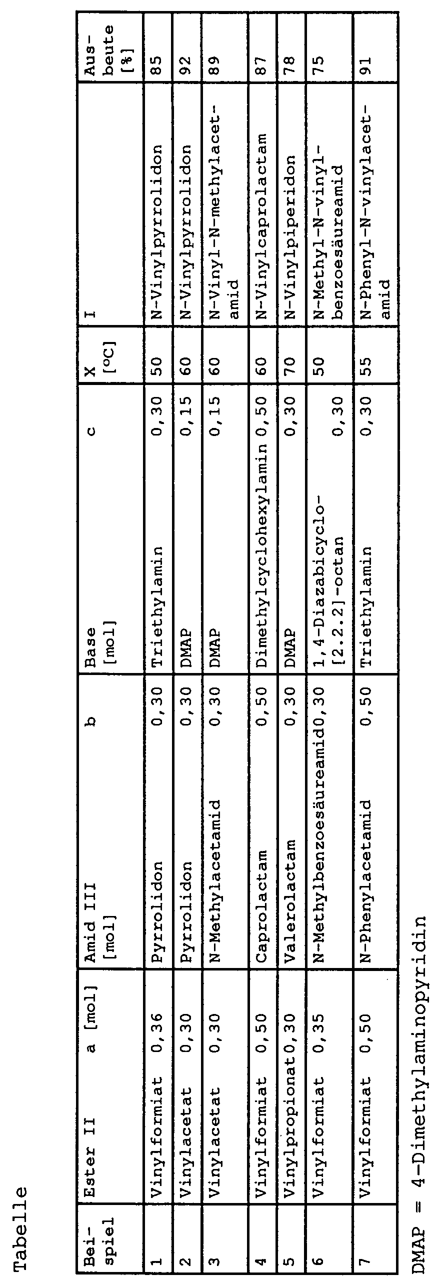

## Patentansprüche

1. Verfahren zur Herstellung von N-Alkenylcarbonsäureamiden der allgemeinen Formel I 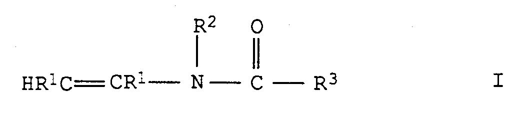 in der mindestens einer der Reste R¹ Wasserstoff bedeutet und der zweite Rest R¹ für Wasserstoff oder eine C₁-C₄-Alkylgruppe steht, der Rest R² für einen aliphatischen, cycloaliphatischen, araliphatischen oder aromatischen Rest steht, der mit dem Rest R³ zu einem 3- bis 10-gliedrigen Brückenglied verbunden sein kann, und der Rest R³ für Wasserstoff, einen aliphatischen, cycloaliphatischen oder aromatischen Rest steht, aus einem Carbonsäurealkenylester der allgemeinen Formel II 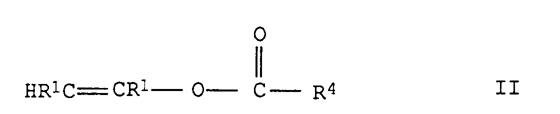 in der R¹ die oben angegebene Bedeutung hat und R⁴ für Wasserstoff, einen aliphatischen, cycloaliphatischen oder aromatischen Rest steht, und einem Carbonsäureamid der allgemeinen Formel III 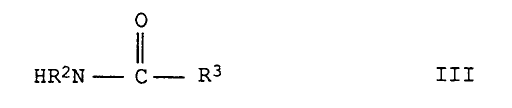 in der die Reste R² und R³ die oben angegebene Bedeutung haben, dadurch gekennzeichnet, daß man die Ausgangsverbindungen in Gegenwart einer Base umsetzt.

2. Verfahren nach Anspruch 1, dadurch gekennzeichnet, daß die Reste R¹ für Wasserstoff stehen.

3. Verfahren nach Anspruch 1 oder 2, dadurch gekennzeichnet, daß der Rest R² für eine C₁-C₂₀-Alkylgruppe steht oder mit dem Rest R³ ein 3- bis 7-gliedriges Alkylenbrückenglied bildet.

4. Verfahren nach den Ansprüchen 1 bis 3, dadurch gekennzeichnet, daß man N-Vinylpyrrolidon oder N-Vinylcaprolactam herstellt.

5. Verfahren nach den Ansprüchen 1 bis 4, dadurch gekennzeichnet, daß man die Umsetzung ohne Lösungsmittel vornimmt.

6. Verfahren nach den Ansprüchen 1 bis 5, dadurch gekennzeichnet, daß man 0,2 bis 1 Äquivalent Base pro Äquivalent Amid der Formel III verwendet.

7. Verfahren nach den Ansprüchen 1 bis 6, dadurch gekennzeichnet, daß man die Umsetzung bei 20 bis 80°C vornimmt.

8. Verfahren nach den Ansprüchen 1 bis 7, dadurch gekennzeichnet, daß man als Base tertiäre Amine verwendet.

9. Verfahren nach den Ansprüchen 1 bis 7, dadurch gekennzeichnet, daß man als Base Natriummethanolat, Kalium-tert.-butylanolat oder Kaliumcarbonat einsetzt.

## Claims

1. A process for preparing N-alkenylcarboxamides of the general formula I 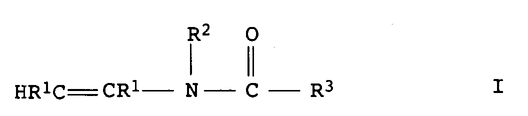 where at least one of the radicals R¹ is hydrogen and the second radical R¹ is hydrogen or a C₁-C₄-alkyl group, the radical R² is an aliphatic, cycloaliphatic, araliphatic or aromatic radical which can be bonded to the radical R³ to give a 3- to 10-membered bridge member, and the radical R³ is hydrogen or an aliphatic, cycloaliphatic or aromatic radical, from an alkenyl carboxylate of the general formula II 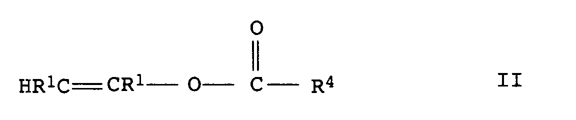 where R¹ has the meanings indicated above and R⁴ is hydrogen or an aliphatic, cycloaliphatic or aromatic radical, and a carboxamide of the general formula III 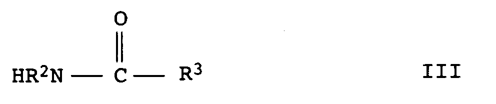 where the radicals R² and R³ have the meanings indicated above, which comprises reacting the starting compounds in the presence of a base.

2. A process as claimed in claim 1, wherein the radicals R¹ are hydrogen.

3. A process as claimed in claim 1 or 2, wherein the radical R² is a C₁-C₂₀-alkyl group or, with the radical R³, forms a 3- to 7-membered alkylene bridge member.

4. A process as claimed in any of claims 1 to 3, wherein N-vinylpyrrolidone or N-vinylcaprolactam is prepared.

5. A process as claimed in any of claims 1 to 4, wherein the reaction is performed without solvent.

6. A process as claimed in any of claims 1 to 5, wherein from 0.2 to 1 equivalent of base are used per equivalent of amide of the formula III.

7. A process as claimed in any of claims 1 to 6, wherein the reaction is performed at 20 to 80°C.

8. A process as claimed in any of claims 1 to 7, wherein the base used is a tertiary amine.

9. A process as claimed in any of claims 1 to 7, wherein the base employed is sodium methoxide, potassium tert-butoxide or potassium carbonate.

## Revendications

1. Procédé de préparation de N-alcénylamides d'acides carboxyliques de formule générale I 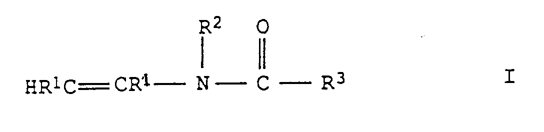 dans laquelle au moins un des restes R¹ représente un atome d'hydrogène et le deuxième reste R¹ est mis pour un atome d'hydrogène ou un groupement alkyle en C₁-C₄, le reste R² est mis pour un reste aliphatique, cycloaliphatique, araliphatique ou aromatique, qui peut être lié avec le reste R³ en un pont à 3-10 maillons, et le reste R³ est mis pour un atome d'hydrogène, un reste aliphatique, cycloaliphatique ou aromatique, à partir d'un ester alcénylique d'acide carboxylique de formule générale II 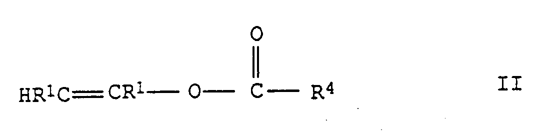 dans laquelle R¹ a la signification donnée ci-dessus et R⁴ est mis pour un atome d'hydrogène, un reste aliphatique, cycloaliphatique ou aromatique, et d'un amide de formule générale III 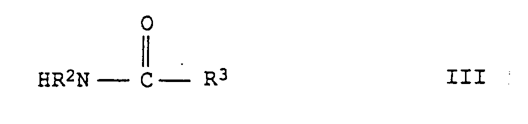 dans laquelle les restes R² et R³ ont la signification donnée ci-dessus, caractérisé en ce que l'on fait réagir les composés de départ en présence d'une base.

2. Procédé selon la revendication 1, caractérisé en ce que les restes R¹ sont mis pour des atomes d'hydrogène.

3. Procédé selon la revendication 1 ou 2, caractérisé en ce que le reste R² est mis pour un groupement alkyle en C₁-C₂₀ ou forme avec le reste R³ un pont alkylène à 3-7 maillons.

4. Procédé selon l'une quelconque des revendications 1 à 3, caractérisé en ce que l'on prépare de la N-vinylpyrrolidone ou du N-vinylcaprolactame.

5. Procédé selon l'une quelconque des revendications 1 à 4, caractérisé en ce que l'on effectue la réaction sans solvant.

6. Procédé selon l'une quelconque des revendications 1 à 5, caractérisé en ce que l'on utilise 0,2-1 équivalent de base par équivalent d'amide de formule III.

7. Procédé selon l'une quelconque des revendications 1 à 6, caractérisé en ce que l'on effectue la réaction à 20-80°C.

8. Procédé selon l'une quelconque des revendications 1 à 7, caractérisé en ce que l'on utilise une amine tertiaire en tant que base.

9. Procédé selon l'une quelconque des revendications 1 à 7, caractérisé en ce que l'on utilise du méthanolate de sodium, du tert.-butanolate de potassium ou du carbonate de potassium en tant que base.
